# EUROPEAN PATENT SPECIFICATION

(11) **EP 2 094 317 B1**
(45) Date of publication and mention of the grant of the patent: **15.09.2010**
(21) Application number: 07817882.9
(22) Date of filing: 08.11.2007
(51) Int. Cl.: A61L 2/18, A61L 2/20

(54) **METHOD FOR MULTI-STEP STERILIZATION**
VERFAHREN ZUR MEHRSTUFIGEN STERILISATION
PROCÉDÉ DE STÉRILISATION EN ÉTAPES

(30) Priority: 08.11.2006 DK 200601453; 08.11.2006 US 857486 P
(43) Date of publication of application: 02.09.2009
(73) Proprietor: B-K Medical ApS, 2730 Herlev (DK)
(72) Inventor: SØRENSEN, Jan, 3460 Birkerød (DK); SIMONY, Carl F., 4180 Sorø (DK); HANSEN, Bo, 2720 Vanløse (DK)
(74) Representative: Nilausen, Kim
(86) International application number: PCT/DK2007/000486
(87) International publication number: WO 2008/055501

(56) References cited:
- WO-A-03/078571
- US-A1- 2002 153 021
- US-A1- 2005 123 436
- US-A1- 2006 199 751
- US-B1- 6 448 062
- US-B1- 6 624 133

## Description

The present invention relates to the field of sterilization of items. More particularly, the invention concerns a method for sterilization.

### Background of the invention

The healthcare segment concerning development of new and more dedicated equipment and tools is growing. Often, these become more and more complex, such as by the use of electronics inside the equipment in order to provide improved tools for examination and treatment. However, a large number of these items is also more fragile, and they often exhibit limited tolerances to elevated temperatures due to a complex material composition and assembly. When dealing with patients, materials and items used during treatment, for example surgery and or examination, often come into direct contact with the patient. When reusing these materials or equipments, they have to be sterilized or disinfected in order to avoid patient to patient contamination.

The most used method for sterilisation is autoclaves, where high temperature, often in combination with steam, is used for sterilization. Often, the temperatures in an autoclave exceed 121 °C or higher, resulting in damage of temperature sensitive equipment.

Therefore, there is a need for alternative sterilization methods for temperature sensitive items. This need is not restricted to the medical sector, and sterility is a necessity in other environments and applications, such as microbiology, military, industrial fermentations, and for food processing areas including slaughter houses.

Other methods are using plasma and/or gas as sterilization agent in combination with vacuum. However, the underpressure or negative pressure generated in these processes will damage equipment with closed cavities, such as ultrasound transducers and endoscopes.

Often, an item or object is considered sterile, when it is free of all living microorganisms, and this state of sterility is the result of a sterilizing procedure. This may be a chemical and/or physical process destroying and/or eliminating all living organisms, which also includes resistant bacterial spores. As it is practically impossible to prove this desired condition, the result of a sterilizing process is defined as a probability of less than one in one million that a microorganism has survived on an item. This is also referred to as "sterility assurance level" and is used by the medical device industry to characterize sterilized medical devices. In practical terms and in view of the current invention, sterilization is defined as a 6 log reduction in microbial load.

Usually, sterilization of objects/items with solid or semisolid surfaces is achieved by surface sterilization. In contrast, liquids and/or gel-like compositions require methods of sterilization with penetrating potential.

Disinfection on the other hand, not to be confused with sterilization, is defined as the process of destroying or inhibiting growth of microorganisms. There are three stages of disinfection, low-level, intermediate-level and high-level disinfection. Low-level disinfection kills vegetative, i.e. growing bacteria, fungi and susceptible viruses, while intermediate-level disinfection kills most bacteria, fungi and viruses, but not bacterial spores. High-level disinfection kills all bacteria, fungi, viruses and may kill bacterial spores, especially when for example prolonged contact times are chosen.

Bacterial spores, or "spores" are considered the most resistant of all living organisms because of their ability to withstand a variety of chemical and/or physical processes, which otherwise are capable of effectively destroying all other living organisms. Fungi have also the ability to produce spores, but in contrast to bacterial spores, fungal spores are less resistant.

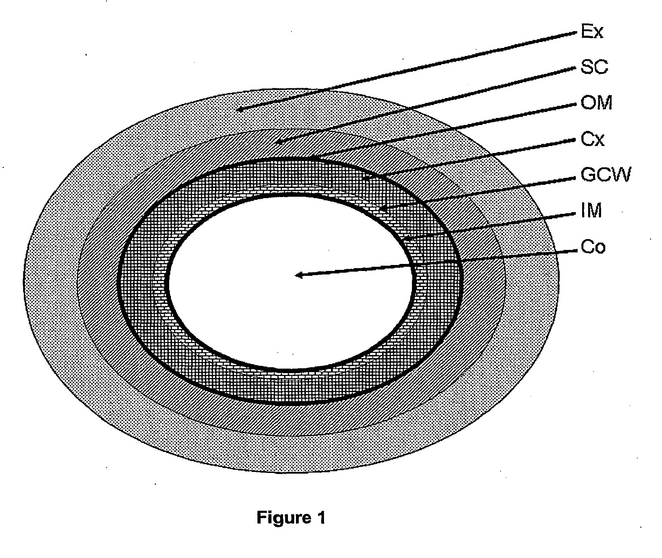

In the following, the current knowledge about the mechanisms involved with respect to resistance of bacterial spores towards sterilization is discussed in more details. Spores are differentiated cells formed within a vegetative bacterial cell in response to unfavourable environmental conditions. It has been reported that spores are several orders of magnitude more resistant to lethal treatments and/or chemical agents than its parent cell. A cross section of a bacterial spore is presented in Fig. 1. The spore is often surrounded by a covering known as the exosporium (Ex), which overlies the spore coat (SC). The spore coat is a complex, multi-layered structure consisting of more than 50. proteins. The spore coat is the major resistance barrier for a large number of chemicals, such as most oxidizing agents including chlorine dioxide, hypochlorite, ozone and peroxynitrite, but not against heat or radiation. The layered outer coats of a bacterial spore are rather inert and play a predominant role in protecting the spore against exogenous agents. It is known that disulfide bridges are a feature of cellular walls and other protein-containing features of bacterial cells. As much as 80% of the total protein of the spore is made up of keratin-like protein. The stability of keratin structures is due to frequent valence cross links (disulfide bonds) and secondary valence cross links (hydrogen bonds) between neighboring polypeptide chains. Keratin-like proteins are resistant to proteolytic enzymes and hydrolysis, but typically insoluble in aqueous salt solutions or dilute acid or base solutions. The cortex (Cx) which consists of peptidoglycan lies beneath the spore coat separated by the outer membrane (OM). The inner membrane (IM) is located between the core wall and the cortex, and surrounds the core (Co) of the endospore. The inner membrane exhibits an extremely low permeability to small hydrophobic and hydrophilic molecules. The core contains normal cell components, such as DNA and ribosomes, but it is considered metabolically inactive.

The mechanisms involved in spore resistance towards sterilization by heat, drying, freezing, toxic chemicals or radiation are not completely understood. The current scientific knowledge is summarized in the recent review article "Spores of Bacillus subtilis: their resistance to and killing by radiation, heat and chemicals") by Setlow (2006; Journal of Applied Microbiology, 101, 514-525.) Setlow summarizes the different factors that contribute to spore resistance towards physical and chemical sterilization. These are (i) significantly reduced water content, (ii) the level and type of spore core mineral ions, (iii) the intrinsic stability of total spore proteins, (iv) saturation and protection of DNA with acid-soluble spore proteins (SASP), (v) DNA repair agents, (vi) alteration in spore DNA photochemistry, (vii) spore coat proteins and, (viii) relative impermeability of the spore inner membrane.

*Bacillus stearothermophilus* is a thermophilic species which can grow at temperatures at 65°C or above. Spores of *Bacillus stearothermophilus* are highly temperature resistant and they are used for example as sterility indicators for steam sterilization. Commercial indicators from FLUKA consist of 1 million spores impregnated on paper strips. These indicators are specified by US military specification MIL-S-35686 and are GMP (Good Manufacturing Practice) requirements of the US FDA (Food and Drug Administration). While *Bacillus stearothermophilus* is mainly used for testing sterilization at high temperatures, *Bacillus atrophaeus* is used as indicator for sterilization at low temperature. The Commercial indicators can be obtained from Raven Biological Laboratories, INC and consist of 1 million spores on stainless steel discs. This microorganism is also part of an US FDA-approved method (FDA Guidance on Premarket notification 510K submissions for Sterilizers intended for use in the health care facilities).

Sterilization procedures can be divided in two major groups, namely physical and chemical processes.

Common physical sterilization procedures are based on heat, either dry heat or moist heat, based on saturated steam, often in combination with pressure. This is for example the major principles in autoclaves, a common instrument used for sterilization.

Heat destroys microorganisms, and their death is caused by denaturation of proteins and enzymes in the cells. This process is accelerated by addition of moisture. Although most vegetative forms of microorganisms are killed in a few minutes at 65°C, certain bacterial spores can withstand temperatures of 115°C for more than 3 hours. It is believed that no living organism can survive direct exposure to saturated steam at 121 °C for more than 15 minutes.

In the absence of moisture, dry heat in the form of hot air requires higher temperatures. Death of microorganisms is again the result of denaturation of proteins and enzymes, combined with oxidation processes. Minimum time requirements for sterilization depend on the temperature used, ranging e.g. from one hour at 171°C or six hours at 121°C.

Radiation may also be used for sterilization and/or disinfection. UV light, form of non-ionizing radiation, is used for sterilization at room temperature, but it is limited to surfaces and some transparent objects. UV is mainly used for sterilizing the interiors of biological safety cabinets between uses. UV is ineffective for sterilization in shaded areas, e.g. cavities or areas under dirt. UV damages many plastics.

Microwave can also be used for sterilization, where the non-ionizing radiation produces energy rich hyperthermic conditions that disrupt life by acting on water molecules, thereby disrupting e.g. cell membranes. Short sterilization cycles of few minutes at only slightly elevated object temperatures can be achieved. Not all objects are suited for sterilization by microwave.

Energy-rich, ionizing radiation in form of β-parties, X-rays or γ-rays is routinely used mainly for batch sterilization, where the ionic energy of the radiation is converted to thermal and chemical energy. Sterilization cycles are long, often overnight. Major advantage is the ability of the ionizing radiation to penetrate through larger objects. Furthermore, dosimetry can be used for immediate assessment of the efficiency of the sterilization process, instead of depending on tedious microbiological tests.

Chemical sterilization procedures are usually applied when the items to be sterilized are heat or moisture sensitive, i.e. when they cannot be sterilized in a dry or steam autoclave. Common chemical agents that are used for killing microorganisms including bacterial spores are ethylene oxide gas, formaldehyde gas, gluturaldehyde activated solution or gas, hydrogen peroxide plasma/vapor, peracetic solution, bleach and ozone gas or in aqueous solution. The major disadvantages of chemical sterilization are (i) the toxicity and (ii) flammability/explosive danger of the chemical compounds used, (iii) the need for aeration of sterilized items after sterilizitation (iv) their often aggressive and corrosive properties towards e.g. plastics and metals, and finally, (v) the long sterilization times needed.

In view of issues related to the use of chemical sterilization and the time requirements of physical sterilization procedures, there is a need for a rapid (< 1 hour, 30 min or less than 30 min), flexible, preferably non-toxic, reliable and simple procedure for sterilizing items that cannot be sterilized by steam, i.e. items that are impaired by temperature or other forms of chemical and/or physical sterilization.

CN 1377708 and CN 1415380 disclose sterilization of an endoscope using enzymes and aqueous ozone.
Denclean 2100 (Damoda aps, DK) is an apparatus for washing and sterilizing rotating dental equipment within 12 min. This is achieved at a maximum temperature of 80 °C, followed by ozone treatment at 80-40 °C and surface sterilization with UV-C.
WO 2005/089819 and US 6 605 260 discloses methods for sterilization based on UV treatment followed by ozone.
WO 2006/079337 discloses an apparatus for sterilization of at least one item with ozone gas and water vapor.
US 6 656 919 discloses a sterilization method based on resuscitating spores followed by germicide treatment.
US 2002/0153021 discloses an automatic washing system, where items are first submitted to a washing process, then are conveyed through a drying-sterilization zone.
US 2005/0123436 discloses a method for abatement of allergens, pathogens and volatile organic compounds.
US 6 624 133 discloses a cleaning product which uses sonic or ultrasonic waves.

### Summary of the invention

The invention concerns a novel method for sterilization, as defined in claim 1, comprising the steps of contacting an item or part of an item with a water-based fluid containing at least one enzyme, and a substantially water-free environment with a gas having oxidative properties.

The invention solves the problem of sterilizing and/or disinfecting items which are prone to be impaired or damaged by for example temperature, pH, positive or negative pressure, radiation, and/or oxidation.

### Detailed description of the invention

The present invention relates to a method as defined in claim 1, which completely or partially satisfies the abovementioned objectives. The method i.a. comprises two distinct steps for sterilization and/or disinfection, where the first step (a) renders a layer or fraction or subfraction of a layer or coat of the microbe or bacterial spore susceptible for the sterilizing/disinfecting action of the second treatment (c).

The inventors surprisingly and unexpectedly discovered that sterilization of items was achieved by treatments with an enzyme containing solution and treatment with a gas containing ozone. This was feasible at temperatures well below the boiling point of water.

The invention relates to a method for sterilization as defined in claim 1 i.a. comprising the steps of contacting one or more item or part of an item with a water-based fluid containing at least one enzyme, and in another distinct step which is performed after the previous step, contacting the one or more item or part of an item with a gas with oxidative properties in a substantially water-free environment. Such steps or treatments may be repeated for maximizing efficiency.

In one embodiment of the invention, treatment with the water-based fluid containing an enzyme comprises ultrasonic treatment, such as sonication, or mixing, vortexing, moving or pumping liquid. In another embodiment of the invention, the ultrasonic treatment/sonication, and/or mixing, vortexing, moving and/or pumping liquid solubilises, dissolves, and/or distributes compounds or particles within said water-based fluid. These compounds and/or particles (including spores, microorganisms and/or dirt) can be removed, or partially removed from the surface of one or more items to be sterilized or disinfected, and said ultrasonic treatment is facilitating this process.

According to the invention, one or more rinsing steps will be between the steps of treatment with a gas with oxidative properties and treatment with a water based fluid containing at least one enzyme. Such one or more rinsing steps may consist of rinsing, flushing or treatment with processed water and flushing with processed gas. The purpose of such a rinsing step can be removal of one or more undesired compounds, including dirt, contamination, chemicals, water, enzyme, detergent, salt, one or more chemicals and the like. This processed water may be demineralised water, tap water or sterile water. Processed water does not need to be sterile if the microorganisms present therein are killed by the subsequent ozone treatment.

By flushing with an appropriate gas, a substantially water-free environment is obtained, especially when the processed gas contains a low level of humidity. A substantially water-free environment can be defined as an environment with a low level of humidity, such as the lumen and/or inner surfaces of a container. This can also refer to a container, its lumen and/or its content, which does not contain visible traces of humidity (such as droplets or pools of water). Such a processed gas may be sterile, sterile filtered, for example dry or dried air, ambient air or nitrogen, and this gas could also comprise ozone. In an embodiment of the invention, the processed gas is ambient air with a low level of humidity. In a further embodiment, the processed gas does not contain any spores or other forms of life that are not killed by the succeeding ozone treatment. In yet another embodiment of the invention, the processed gas has a lower level of humidity than ambient air. This dry or dried air provides a substantially water-free environment according to the invention.

The water-based fluid applied in the present invention contains an enzyme or several enzymes selected from the group comprising microbial cell wall modifying or degrading enzymes, protein modifying or degrading enzymes and/or fat modifying or degrading enzymes. Without limitation, these enzymes can be selected from the group comprising cellulases, chitinases, amylases, proteases, lysozyme(s), phosphatases, kinases and/or lipases. In an embodiment of the invention, the enzyme or enzymes are capable of degrading the cell wall of bacterial spores. More precisely and non exclusively, the enzyme or enzymes are capable of degrading or impairing at least in part the exosporium, the spore coat, the outer membrane, the spore coat and/or the inner membrane. It is believed that these enzyme or enzymes are capable of impairing the spore and/or the protective layer(s) of the spores to such a degree, that the subsequent treatment with a gas containing ozone results in sterilization and/or disinfection according to the invention.

In a further embodiment of the invention, the enzymatic treatment comprises incubation with one or more enzymes selected from the group consisting of cellulose; chitinase; amylase; protease; lysozyme; phosphatase; kinase; lipase; sulfatase (a type of esterase enzyme which removes sulfate from a variety of substrates. Ex. Galactosamine-6 sulfatase, N-acetylglucos-amine-6-sulfatase), neuraminidase (a glycoside hydrolase enzyme (EC 3.2.1.18). It is frequently found as an antigenic glycoprotein and is best known as one of the enzymes found on the surface of the Influenza virus), aminopeptidase (a zinc-dependent enzyme produced by glands of the small intestine and assists in the enzymatic digestion of proteins therein), a digestive enzyme produced by a gland of a mammal, such as dipeptidase, maltase, sucrase, lactase, and enterokinase; achromopeptidase (a lysyl endopeptidase with a MW of ∼27kD. It is useful for lysis of Gram-positive bacteria that are resistant to lysozyme); lysostaphin (a Staphylococcus simulans metallo-endopeptidase. It can function as an antimicrobial against Staphylococcus aureus. Lysostaphin is a zinc endopeptidase with a molecular weight of approximately 25 kDa. Because lysostaphin cleaves the poly-glycine crosslinks in the peptidoglycan layer of the cell wall of Staphylococcus species it has been found useful for cell lysis); labiase from Streptomyces fulvissimus is an enzyme preparation useful for the lysis of many Gram-positive bacteria such as Lacto-bacillus, Aerococcus and Streptococcus. Labiase contains β-N-acetyl-D-glucosaminidase and lysozyme activity); mutanolysin provides gentle cell lysis for the isolation of easily degradable biomolecules and RNA from bacteria. It has been used in the formation of spheroplasts for isolation of DNA. Mutanolysin is a 23kD N-Acetyl Muramidase, like lysozyme, is a muralytic enzyme that cleaves the N-acetylmuramyl-β(1-4)-N-acetylglucosamine linkage of the bacterial cell wall polymer peptidoglycan-polysaccharide. Its carboxy terminal moieties are involved in the recognition and binding of unique cell wall polymers. Mutanolysin lyses Listeria and other gram positive bacteria such as Lactobacillus and Lactococcus); endochitinase A (an enzyme that breaks down glycosidic bonds in chitin. It is found in chitinivorous bacteria (bacteria that are able to digest chitin); chitobiosidase (another chitin degrading enzyme); N-Acetyl-beta-Glucos-aminidase; hyaluronidase (Chondroitin); chondroitinase (ABC, AC or C); cysteine proteases have a common catalytic mechanism that involves a nucleophilic cysteine thiol in a catalytic triad. The first step is deprotonation of a thiol in the enzyme's active site by an adjacent amino acid with a basic side chain, usually a histidine residue. The next step is nucleophilic attack by the deprotonated cysteine's anionic sulfur on the substrate carbonyl carbon. In this step, a fragment of the substrate is released with an amine terminus, the histidine residue in the protease is restored to its deprotonated form, and a thioester intermediate linking the new carboxy-terminus of the substrate to the cysteine thiol is formed. The thioester bond is subsequently hydrolyzed to generate a carboxylic acid moiety on the remaining substrate fragment, while regenerating the free enzyme. Ex. Papain, Cathepsins, Caspases, Calpains); caspases: As proteases, they are enzymes that cleave (cut) other proteins. They are called cysteine proteases, because they use a cysteine residue to cut those proteins, and called caspases because the cysteine residue cleaves their substrate proteins at the aspartic acid residue; Germination protease (GPR; an atypical aspartic acid protease located in spore coats); aspartyl protease (a protease which utilizes an aspartic acid residue for catalysis of their peptide substrates. They typically have two highly-conserved aspartates in the active site and are optimally active at acidic pH. Nearly all known aspartyl proteases are inhibited by pepstatin. Ex. HIV-1 protease - a major drug-target for treatment of HIV, chymosin (or "rennin", Renin (with one "n"), cathepsin D, pepsin, plasmepsin, aspartic protease precursor pepsinogen); keratinase and or keratanase.

In the context of this invention, the term "spore" or "spores" relates to bacterial spore or spores unless stated otherwise. However, in one embodiment fungi, moulds and/or fungal spores are sterilized or disinfected according to the present invention.

In one embodiment, the water-based fluid, in addition to enzyme(s), contains suitable additives, such as salts, coenzymes, trace elements, stabilizing agents, buffering agents, polar and nonpolar detergents as well as antimicrobial agents. In a further embodiment, the enzyme or enzymes can be provided as a composition for example as powder or tablet, comprising 30% or more phosphate; 5-15% bleaching agents and less than 5% enzyme (percentages are weight percent in relation to the total weight of the composition). In another embodiment, the enzyme or enzymes can be provided as a composition, for example as powder or tablet, comprising 15-30% phosphate(s), less than 5% bleaches with oxygen, less than 5% nonionic tensides; less than 5% perfume (for example limonene),and than 5% enzyme (percentages are weight percent in relation to the total weight of the composition). In another embodiment, the water-based fluid containing at least one enzyme is provided by dissolving a composition, for example as powder or tablet, comprising 30% or more phosphate, 5-15% bleaching agents and less than 5% enzyme; or 15-30% phosphate(s), less than 5% bleaches with oxygen, less than 5% nonionic tensides; less than 5% perfume (for example limonene),and than 5% enzyme (percentages are weight percent in relation to the total weight of the composition). In yet another embodiment of the invention, the water-based fluid comprising an enzyme is provided by diluting a concentrated stock solution. In yet a further embodiment, the water-based fluid comprising an enzyme is provided by dissolving a composition used for or suitable for household dishwashers.

In another embodiment of the present invention, the enzyme or enzymes are dissolved or suspended in a water based fluid, which is applied in the form of cold steam. vapor and/or as spray. According to the invention, in such an embodiment the water-based fluid comprising an enzyme and or other compounds can be present and applied in droplets, which can be comparable (e.g. in size) to droplets such as in steam or mist, or droplets produced by an atomizer known in the art. The steam/mist/vapor/spray is likely to condensate and/or accumulate on surfaces, such as surfaces of an item to be sterilized according to the invention. Thereby a film of liquid, preferably a continuous film, is generated. Cold steam indicates also, that this "steam" is not hot, e.g. near or even above the boiling point of water, but at a significantly lower temperature, such as the temperatures provided according to the current invention.

The invention is not limited to a single, defined composition used for each step, and may be considered flexible and modular in terms of providing several different sterilization or disinfection protocols, according to the item or group of items to be sterilized, as well as depending on the microbial load, the time span available for sterilizing, tolerances towards various chemicals, temperatures, pressures, sizes and volumes which have to be treated.

Sterilization or disinfection according to the invention is a 4 log reduction of microorganisms, spores or microbial burden, more preferably a 5 log reduction and most preferably a 6 log reduction or even more. Sterilization is at least a 6 log reduction, disinfection at least a 4 log reduction, preferably 5 log reduction. Unless stated otherwise, sterilization or disinfection in the context of the present invention relates to surface sterilization or disinfection, respectively.

Another important feature of the present invention is the absence of higher temperatures during the sterilization or disinfection process. Preferably, the maximum surface temperature on the item to be sterilized or disinfected is below 100°C, preferably below 50°C, more preferably below 37°C, and most preferably around room temperature 20°C. The appropriate temperature or temperatures to be selected for the different steps of the invention depend on the efficiency of the different agents used in each step, combined with the temperature sensitivity of the objects or items to be sterilized, the desired level of disinfection (high, medium or low) and the total time available for the combined treatment.

A selected pH or change or changes of pH may also be accomplished. The pH of the water-based fluids, including processed water, may be in the range of pH 2 to 12, preferably 4 to 10, more preferably 6 to 8 and most preferably 6,8 to 7,2. The pH may be constant, or it may change during the treatment. The pH of the various fluids used during sterilization or disinfection may be similar, or very different. In one embodiment, the pH is in the area of the enzyme's pH optimum

Likewise, different pressures (positive or negative) may be applied during the invention. In one embodiment, the absolute pressure applied is in the range of 1 to 300 kPa, preferably 10 to 200 kPa, more preferably 50 to 150 kPa, and most preferably 80 to 120kPa.

The method according to the invention allows for a rapid sterilization, and the total time needed for sterilization is not exceeding 60 min, preferably 30 min, more preferably 15 min, and most preferably 5 min. These time spans comprise the processing steps of contacting an item or part of an item with a water-based fluid or other liquids or gas containing at least one enzyme, and in another distinct step, contacting the item or part of an item with a gas with oxidative properties in a substantially water-free environment. For many applications, the total time needed for treatment with one or several water-based fluids is not exceeding 30 min, preferably 10 min, more preferably 5 min, and most preferably 2 min.

Likewise, in the view of a rapid method for sterilization and/or disinfection, the total time needed for treatment for contacting the item or part of an item with a gas with oxidative properties in a substantially water-free environment does not exceed 20 min, preferably 10 min, more preferably 5 min, and most preferably 1 min. However, once treated according to the invention, the sterile items may be left in a gas atmosphere for storage until they are needed.

The gas with oxidizing properties which is used according to the invention is selected limitations from the group containing oxygen, ozone, ethylene oxide, hydrogen peroxide.

In an embodiment of the invention, ozone is used as gas with oxidizing properties in a substantially water-free environment. Ozone concentrations are in the range of 1 to 10000 ppm, preferably 5 to 100 ppm, more preferably 10 to 60 ppm, 40-60 ppm or 30-50 ppm, and most preferably 15-30 ppm. The ozone concentration applied is dependent on the nature and degree of biological contamination, as well as the susceptibility to oxidative damage of the object to be sterilized.

Ozone in gas decays to O₂ with a half-life of approximately 3 days at 20 °C, but as fast as 1.5 seconds at 250 °C. This process may be accelerated by the use of known catalysts. Ozone may also be dissolved in water, where its half-life is considerably shorter, e.g. approximately 30 min at 15 °C or 8 min at or 35 °C at pH 7. Decomposition is faster in a basic environment, e.g. 3 min at pH 10.4 at 15 °C. Ozone may also be converted to oxygen by means of UV light. from the group comprising laboratory items, medical items, dental items, veterinary items, military items, biological items and/or food processing related items. Furthermore, the method according to the invention may be used for satellites, space rockets and the like, where contamination of space, planets, asteroids, comets has to be avoided, for example in the context of investigating the question of presence or absence of extraterrestrial life forms.

Items or objects or parts thereof that are suitable for sterilization/disinfection are those that would be impaired by temperature, pH, pressure, radiation and/or oxidation if subjected to other forms of sterilization/disinfection. Such items to be sterilized or disinfected may be selected from the group comprising laboratory items, medical items, dental items, military items, biological items, and/or food processing related items.

Such items or parts thereof could be selected from the group of medical instruments including instruments used for medical procedures in humans or animals including dental instruments. In one embodiment of the current invention, endoscopes and/or ultrasound transducers are sterilized.

Often, these items will be reusable items, but the invention is not limited to reusable items only, and disposable items may be processed as well by the use of the milder and gentler method of sterilization/disinfection according to the invention. For example, disposable items could be packaged/wrapped in a container or foil in an ozone containing atmosphere.

Another use of the method according to the invention is application during organ transplantation, such as surface treatment of tissues or organs of human or animal origin prior to implantation. A further use relates to sterilization or disinfection of one or more implants comprising pacemakers, joints, e.g. artificial hips, knees and the like, ligaments, bones, limbs or the like.

The method according to the invention is also suitable for decontamination of items, parts of items or surfaces, e.g. in the military context of fighting microbial warfare or after terrorist attack or suspicion of terrorist or military activities comprising microbial activities.

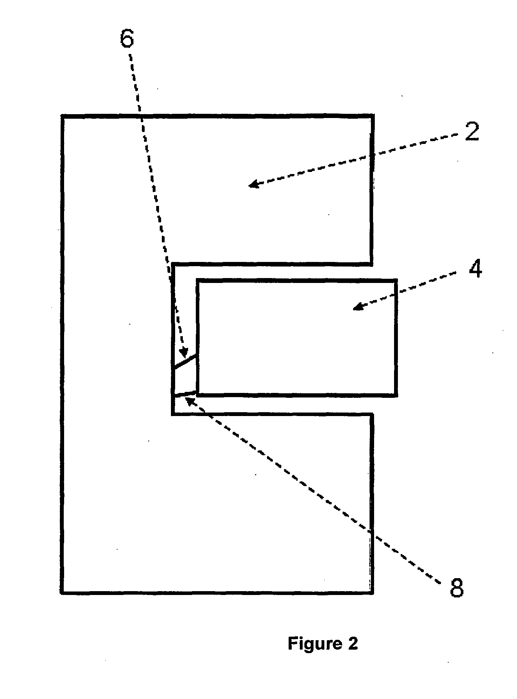

An apparatus comprising the necessary means for performing the above mentioned method may be applied (Fig. 2). In one embodiment a single, combined apparatus is used for performing steps of rendering a layer or fraction or subfraction of a layer or coat of the bacterial spore susceptible for the sporicidal/sterilizing action of the second treatment. Another embodiment is not limited to a single apparatus. Different devices may be used for the different steps according to the invention. For example, the rinsing and/or incubations step(s) could be performed in using one specialized apparatus, while the ozone treatment could be carried out in another device, and the time intervals in between the different steps may be selected accordingly.

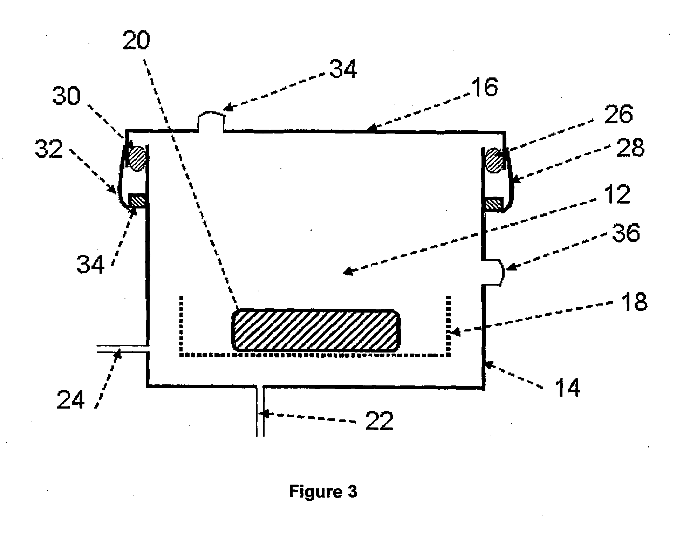

Such an apparatus may also comprise one or more separate containers for sterilizing one or more items (Fig. 3). Such a container may be removed from said apparatus, and more preferably, the content of such a container will remain sterile upon removal from said apparatus. If appropriate, the whole container, or just a part of it, for example the device (18) in Fig. 3 on which the item to be sterilized (20) rests, may be used in the transportation from one place to another, such as from one apparatus to another apparatus for performing one or more of the following treatments: a washing step, an incubation step with an aqueous fluid comprising at least one enzyme, a rinsing step with processed water, a rinsing step with processed gas and/or a step comprising treatment with an oxidizing gas.

In an embodiment according to the invention, a pressure higher than ambient pressure is maintained in the container. Such a container may be equipped with a device or indicator of sterility, in an either manual, semi- or fully automated fashion. Such a container may comprise a pressure indicator, indicating that the content of said container is sterilized.

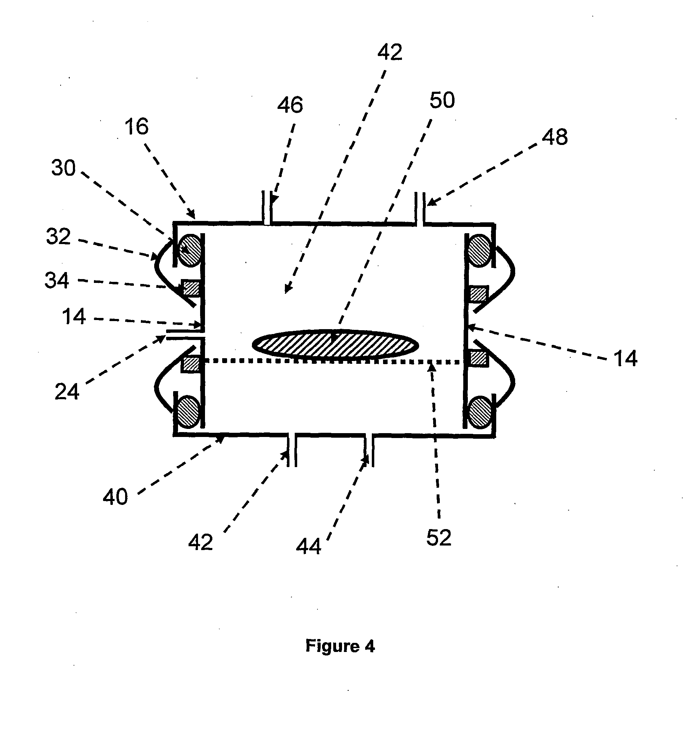

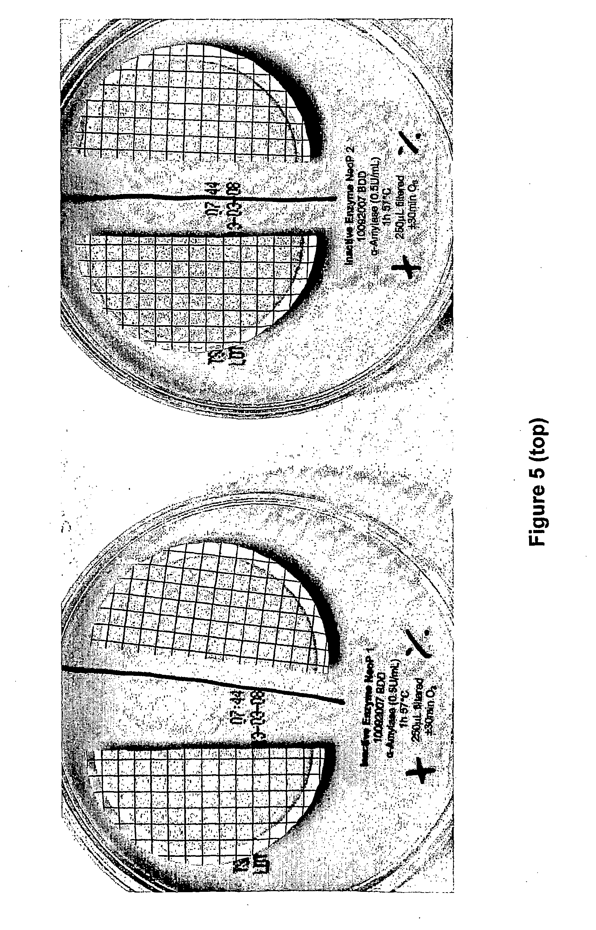

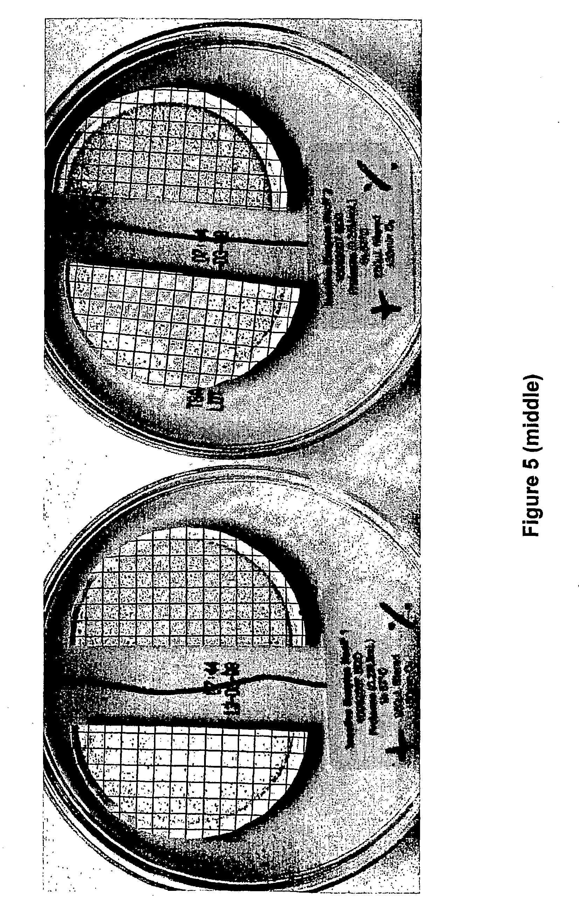

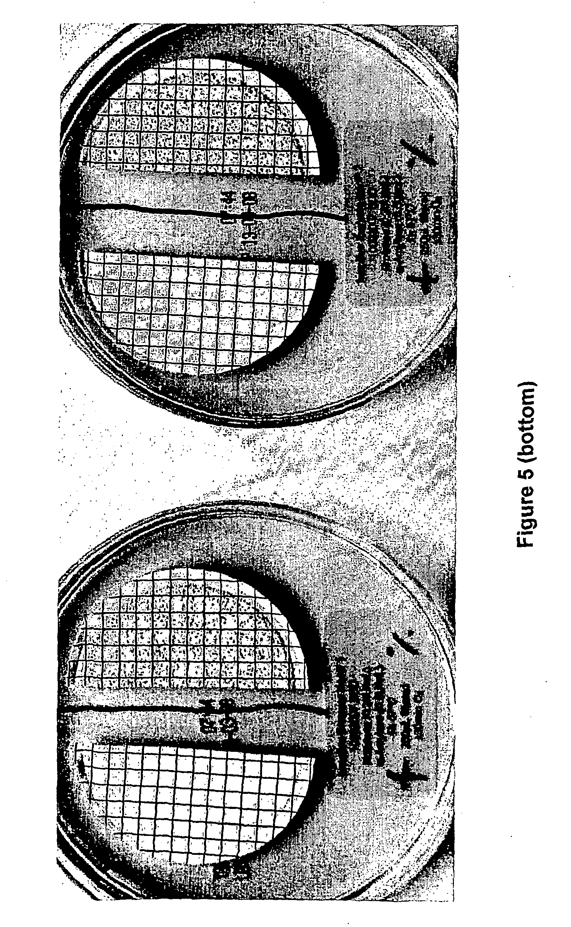

An alternative embodiment of a container according to the invention is illustrated schematically in Fig. 4. The container for sterilization and/or disinfection consists of a body (14) which is closed with two lids (16, 40). The lumen of the container is held air- and water tight by a seals, and clamping devices. The item or items to be treated (50) rest on a device (52), which is attached to the body (14). By removing both lids, one or several rinsing/washing/incubation steps can be performed, giving access to for washing/rinsing/treatment from top and bottom, if desired. Alternatively, the object/device to be treated, while situated on the device (52) surrounded by the body (14), may be placed in a rinsing/washing/incubation device. Once the lids are closed, the lumen (42) of the device can be filled with liquids and gasses via one or more inlets, outlets or in-and outlets (24, 43, 44, 46, 48), which can be situated either on the one or the other lid or both, or on the body (14).

### Brief description of the drawings

**Figure 1****:**
   Schematic representation of a bacterial spore and the complex spore coat, not drawn to scale, modified from Setlow (2006). Note that the sizes of the layers of a spore may vary considerably between species. Ex: exosporium; SC: spore coat; OM: outer membrane; Cx: Cortex; GCW: germ cell wall; IM: inner membrane; Co: Core.
**Figure 2****:**
   Schematic representation of an apparatus (2) with a sterilizing chamber (4), one or more inlets (6) and one or more outlets (8).
**Figure 3****:**
   Schematic representation of a sterilizing chamber A lumen (12) is created between the body of the sterilizing chamber (14) and a removable lid (16). Lid (16) and body (14) remain air-tightened by use of a seal (26, 30) and clamping device (28, 32, 34). The object to be sterilized (20) rests on a device (18), which may be detachable from the body (14). Liquids and gases are according to the invention transported into and out of the lumen (12) by one or more inlets and outlets (22, 24). A pressure indicator located on either lid (34) or body of the sterilizing chamber (36) indicates if there is an overpressure present, indicating sterility of the item to be sterilized (20).
**Figure 4****:**
   Schematic representation of an alternative disinfection/sterilizing chamber. A lumen (42) is created between two lids (16 and 40) and the body of the sterilizing chamber (14). As in Fig. 3, the container is air- and water tightened by seals (30) and one or more clamping devices (32, 34). For simplicity, not all of the depicted seals and clamping devices are numbered. The item or object to be sterilized or disinfected (50) rests on a device (52), which is attached to the body (14). By removing both lids, one or several rinsing/washing/incubation steps can be performed, giving access to washing/rinsing/treatment from top and bottom if desired. The lumen (42) of the device can be filled with liquids and/or gasses via one or more inlets, outlets or in-and outlets (24, 43, 44, 46, 48), which can be situated either on the one or the other lid or both, or on the body (14). In the present example, only of one of the potentially several in/outlet(s) (24) are shown.
**Figure 5****:**
   Photographs showing colony growth on filter paper +/- ozone illustrating treatment with α-amylase (top), protease (middle), α-amylase and protease (bottom). Experiments were performed in duplicate (left vs. right petri dish). "+" indicates a filter half treated with ozone; "%" indicates filter half not treated with ozone. Further details are provided in Example 7 (3).

### Examples

### EXAMPLE 1

Experiments which failed to provide a gentle, rapid disinfection. Bioburden reduction was tested by using a commercial stainless steel disk with 1-2 million spores of *Bacillus atropheus* (Raven Biological Laboratories) according to the manufacturer's protocol. Similar disks can be obtained from several suppliers, and such disks are known in the art, as they are used e.g in US-FDA approved methods for testing sterilizers intended for use in health care facilities. Standard procedures were modified accordingly. Unless stated otherwise, such disks where used in the present and the following examples as standardized and defined test item and/or source of spores.
(A) The initial test using humid air with more than 80 % rel. humidity at a temperature at 55°C to 60°C and a ozone concentration of 20 PPM. This process did not reduce the bio burden significantly, as 400000-700000 cfu survived.
(B) Experiment with water containing 10 ml of 4 mM L-alanine as substrates for resuscitating the spores in order to make susceptible for ozone treatment revealed that this method was too time consuming (several hours) for a rapid and reliable sterilization process (even 24 h where not sufficient); 700 -1500 cfu survived, which is equivalent to a 3 log reduction of bio-burden.
(C) Two ml of a detergent (Barsol 4%) were added to 10 ml of demineralized water and sonication for 30 minutes. Thereafter, the disc was flushed with demineralized water at 25°C for 10 seconds and dried with air (∼50% relative humidity) at 20-25 °C for 10 seconds. The dried object was treated with ozone in sterile filtered ambient air (∼50% relative humidity at room temperature) at a concentration of 30-50 ppm for a period of 15 minutes at room temperature. This treatment reduced the bioburden by 3 logs (500-1000 cfu survived).

### EXAMPLE 2

Rapid and non-corrosive sterilization was achieved by submerging a stainless steel disk with *Bacillus atropheus* spores from Raven Biological Laboratories in an aqueous solution containing enzymes at 50 °C. This solution was obtained by dissolving 4 g/I of a commercially available tablet for household dishwashers purchased from Dansk Supermarked Indkøb A/S), containing more than 30% phosphate, 5-15% bleaching agents and less than 5% of proteases and amylases. The solution, in which the steel disk was completely submerged, was sonicated in an ultrasonic bath for 15 minutes in order to provide mixing of the fluid comprising the steel disk. Vortexing at 250-350 rpm on a shaker table could be used as well in order to provide a desired mixing effect. Thereafter, the object was flushed with demineralized water at 25°C for 10 seconds and dried with air (∼50% relative humidity) at 20-25°C for 10 seconds. The dried object was treated with ozone in sterile-filtered ambient air (∼50% relative humidity at room temperature) at an ozone concentration of 30-60 ppm for a period of 15 minutes at room temperature ("OBOX"; see following examples for further details on ozone-treatment). This treatment reduced the bio-burden with 6 log (< 10 spores), while the control experiment without ozone revealed a significant number of spores remaining on the carrier 500-1000 cfu. Bio-burden reduction was tested as described in Experiment 1. The result <10 cfu indicates that less than 10 cfu are found according to the analysis method.

### EXAMPLE 3

A material compatibility test was performed to investigate ozone's corrosivity with respect to materials commonly used for medical equipments, such as ultrasound transducers and flexible endoscopes. Following materials were selected as representatives of different groups of materials: ABS polyurethane TPX, silicone, neoprene rubber and PVC. Materials were tested in a chamber with 40 to 50 ppm (parts per million) ozone at 25°C to 40°C for 80 hours subjected to cycles of one hour in ozone and one hour in air. There were no visible damages or changes to the surface of the material compared to untreated control samples.

### EXAMPLE 4

### Purpose/Scope

Determination of the effect of treatment of spores of Bacillus atrophaeus with Phospholipase A₂ (PLA₂), Glucose Oxidase (GO) and α-Chymotrypsin (αCT) at 37°C, followed by subsequent of ozone-treatment.

### Materials and Methods

PLA₂ Buffer (Bf): 3.675mL 1M NaCl (147mM); 117.5µL 1 M CaCl₂ (4.7mM);
21.208mL sterile H₂O (pH 8.03)
PLA₂: 10mg/mL in sterile H2O (211 U/mg; 2.11 U/µL)
GO Bf: 45mM Sodium Acetate; 0.01% Saccharose solution (pH 5.10);
10% D(+)-Glucose (se below)
GO: 100mg/100mL 1mM HCl; 20mM CaCl₂ (93U/mg; 0.5U/µL)
αCT Bf: 0.1 M Tris-HCl; 10mM CaCl₂; (pH 7.82)
αCT: 10mg/mL in 1 mM HCl (98.7U/mg; 0.987U/µL)

(1) αCT: To each of two 2-mL cryo tubes, 1995.0µL αCT Bf is added followed by 5µL 0.987U/µL αCT. Immediately, a Bacillus atrophaeus (BA)-inoculated disc is placed in each tube, the tube is closed and turned up-side-down two-three times. The duplicate sample is incubated at 37°Cfor ∼1hour under vortexing at ∼300rpm (actual time: 1h 5 min). Two sterile filter units are mounted according to instructions and filled with 12.5mL sterile H₂O; then the duplicate sample is retrieved from the incubator and 250µL sample fluid is added to the filter unit. Vacuum is applied and within ∼30s the fluid is gone. The vacuum is stopped and another 12.5mL sterile H2O is added to the filter unit before re-applying the vacuum. This is repeated once more. The filter unit is labelled and dismounted from the filtering system and put aside for later processing.
(2) PLA₂: To each of two 2-mL cryo tubes, 1999µL PLA₂ Bf is added followed by 1 µL 2.11U/µL PLA₂. Immediately, a Bacillus atrophaeus (BA)-inoculated disc is placed in each tube, the tube is closed, and is turned up-side-down two-three times. The duplicate sample is incubated as in (1) (actual time1h 20min). The subsequent processing is carried out similar to (1).
(3) GO: To each of two 2-mL cryo tubes, 1790µL GO Bf are added, 200µl 10% D(+)-Glucose, followed by 10µL 0.5U/µL GO. Immediately, a Bacillus atrophaeus (BA)-inoculated disc is placed in each tube, the tube is closed, and turned up-side-down two-three times. The duplicate sample is incubated as in (1) (actual time 1h 26 min). The subsequent processing is carried out similar to (1).

(1-3) The filter unit is disassembled aseptically. The lid is discarded and the funnel is bend midways for removal and discarded, so the filter can be extracted with a sterile tweezers and cut in two halves with a sterile scissor. The filter holder is discarded also. One half of the filter is placed on a sterile watch glass, the filter is divided schematically (duplicate 1 and 2) and labelled with ID. The other half is placed on a sterile Tryptic Soy Agar (TSA) plate - one for each duplicate -, which is likewise divided schematically and labelled with ID (and denoted ±O₃). No air bubbles permitted between the filter and the agar!
   Ozone treatment is performed by placing the sample (e.g. watch glass with filter or filter halves from duplicate experiments) in a -20 I chamber ("OBOX") for 15min (interval: 8s pause, followed by 52s O₃, thereby providing 40-60 ppm ozone); the chamber is flushed with ozone containing air (60 I/min). This "OBOX" was also used in the other Experiments, with the same or similar conditions, unless stated otherwise.
   The ozone-treated filter halves are placed on the TSA plate next to their corresponding untreated half and incubated overnight at ∼35°C, while placing a bowl of water in the bottom of the oven, to maintain an appropriate relative humidity, and to avoid drying out the agar. The following day the number of cfu is counted. Results of such experiments are presented in Table 1.

**Table 1. Effects of treatment with α-Chymotrypsin, Phospholipase A₂ (PLA₂), or Glucose Oxidase (GO) +/- ozone (O₃).**

| | Enzyme without O₃ | Enzyme and O₃ |
|---|---|---|
| α-Chymotrypsin | 8320 cfu | 7020 cfu |
| Phospholipase A₂ | 6240 cfu | 4160 cfu |
| Glucose oxidase | 8060 cfu | 6240 cfu |

| | | |
|---|---|---|
| cfu = total number of spores/cfu in sample. | | |

Conclusion: Treatment with either α-Chymotrypsin, Phospholipase A₂ (PLA₂), or Glucose Oxidase (GO) showed an approximate 3 log reduction in cfu compared to untreated samples. The total number of spores/cfu on the metal disks was 1 x 10⁶ spores. Generally, ozone treatment reduced the number of cfu compared to the untreated samples.

### EXAMPLE 5

The aim of this study was to determine and compare, whether spores of *Bacillus atrophaeus* respond to incubations with various enzymes. Thereto, the number of bacterial colonies (cfu) was determined on appropriate agar plates after enzyme treatment.

*Bacillus atrophaeus* spores are highly resistant to heat and chemicals and their use are officially recognized for sterilization procedure certification. Novel sterilization procedures are needed for medical equipment containing sensitive materials that do not tolerate high temperatures and hence can not be sterilized by use of heat. Here 8 different commercially available enzymes are investigated for a possible effect on spore viability.

### Materials and Methods

### Bacillus atrophaeus spores:

Stainless steel discs with *Bacillus atrophaeus* spores (log 6, cat# 1-6100ST, Raven Biological Laboratories).

### Enzymes:

Phospholipase A_{2;} Hog pancreas; *00299 FLUKA*
Protease; Bacillus sp.; *P5985 Sigma*
Keratanase (β-Endo-galactosidase); Recombinant bacteroides fragilis);
*G6920 Sigma*
Trypsin; Hog pancreas; *93613 FLUKA*
α-Amylase; Bacillus subtilis;*10070 FLUKA*
α-Chymotrypsin; Bovine pancreas; *27270 FLUKA*
Glucose Oxidase; Aspergillus niger; *49178 FLUKA*
Lysozyme; Chicken Egg White; *L7651 Sigma*

### Medium:

Nutrient Agar (i.e. Peptone, Meat extract and Agar).

### Procedure:

Spores and enzymes were incubated in buffer solutions as described in Appendix 1. The mixtures were incubated at 37°C for 3-5 hours and then stored at 4°C over night. Next day dead cells were added and the sample centrifuged, diluted and plated as described in Appendix 1. As dead cells an *E. coli* culture (strain DH5a) treated with 1 % glutaraldehyde for 15 min at 37°C were used. An initial test using these dead cells showed that similar spore colony numbers were found before and after the centrifugation procedure. Consequently, the purpose of the dead cells, to form a visible pellet and to ensure a good spore recovery, was fulfilled.

Plates were incubated at 30°C for 1-2 days and the number of *Bacillus atrophaeus* colonies counted. The results are shown in Appendix 2.

### Results:

For 7 of the 8 enzymes tested a similar reduction in number of colonies were found by plating spores treated with both a high and a low concentration of the enzyme. Glucose oxidase without addition of glucose differed, where the low enzyme concentration gave a low colony count compared to the high enzyme concentration. In addition, glucose oxidase with the addition of glucose produced a colony count below the detection limit of the experiment. The results are summarized in Table 2. Please refer to Appendix for further details.

**Table 2: Summary of experimental results.**

| **Enzyme** | **Colony count (100 µl)** | **% survival** |
|---|---|---|
| Lysozyme | 91 | 47% |
| Keratanase | 75 | 39% |
| Protease | 91 | 47% |
| Alfa-amylase | 144 | 74% |
| Trypsin | 86 | 44% |
| Alfa-chymotrypsin | 124 | 64% |
| Glucose oxidase (P) | 0 | 0% |
| Glucose oxidase (M-H) | 110 | 56% |
| Glucose oxidase (M-L) | 7 | 3% |
| Phospholipase A2 | 121 | 62% |
| Direct plating | 195 | 100% |

### Preparation of spore suspension:

To a sterile tube 10ml buffer is added.
Addition of 5 *Bacillus atrophaeus* discs.
Liberation and suspension of spores by turning the tube up and down five times.
Concentration: 5x10⁵ spores/ml
The same stock solution is utilized for the entire series of experiments

### General:

Enzyme analysis is prepared in 1.5-ml eppendorf tubes (3 identical samples = triplicates).
Incubation 37°C for 15-20hours (o/n incubation).
Addition of 200µl dead cells, mix and centrifuge (20.000g for 5min).
Pellet is resuspended in 1ml sterile water.
Preparation 20x dilution (50µl resuspension + 950µl sterile water).
Plating 100µl and 50µl dilution on Nutrient Broth agar plates.

Dilution leads to app. 250 and app. 125 spores/colonies per plate.
(Utilization of altogether 12 plates per enzyme test).
Incubation of plates at 30°C and counting of colonies after incubation overnight (o/n).

### 1. Lysozyme.

Preparation 10mg/ml solution of lysozyme in STET buffer
(STET buffer: 8% sucrose, 5% TritonX-100, 50mM Tris pH8.0, 50mM EDTA)
Enzyme analysis in triplicate of 2 concentrations of lysozyme (*High* and *Low*)

| | | | |
|---|---|---|---|
| *High* | 800µl STET buffer | *Low* | 800µl STET buffer |
| | 100µl spore suspension | | 100µl spore suspension |
| | 100µl lysozyme solution | | 10µl lysozyme solution |
| | | | 90µl sterile water |

Incubation and plating as described in the general section.

### 2. Keratanase.

Preparation 100mM acetate buffer med pH6.0
(0,41 g Na-acetate in 50ml sterile water, pH is adjusted to 6.0 with NaOH/HCl)
Enzyme is drawn directly from tube (app. 10µl left)
Enzyme analysis in triplicate of 2 concentrations of keratanase (*High* and *Low*)

| | | | |
|---|---|---|---|
| *High* | 900µl acetate buffer | *Low* | 900µl acetate buffer |
| | 100µl spore suspension | | 100µl spore suspension |
| | 5µl keratanase enzyme | | 1µl keratanase enzyme |

Incubation and plating as described in the general section.

### 3. Protease.

Preparation 10mM Tris pH8.0
(99ml sterile water + 1 ml 1M Tris pH8.0)
Enzyme is drawn directly from tube (liquid product)
Enzyme analysis in triplicate of 2 concentrations of protease (*High* and *Low*)

| | | | |
|---|---|---|---|
| *High* | 800µl Tris buffer | *Low* | 800µl Tris buffer |
| | 100µl spore suspension | | 100µl spore suspension |
| | 100µl protease enzyme | | 10µl protease enzym |
| | | | 90µl sterile water |

Incubation and plating as described in the general section.

### 4. α-amylase.

Preparation 50mM Tris pH8.0
(95ml sterile water + 5ml 1M Tris pH8.0)
Preparation 10mg/ml solution of α-amylase enzyme in Tris buffer
Enzyme analysis in triplicate of 2 concentrations of α-amylase (*High* and *Low*)

| | | | |
|---|---|---|---|
| *High.* | 800µl Tris buffer | *Low* | 800µl Tris buffer |
| | 100µl spore suspension | | 100µl spore suspension |
| | 100µl α-amylase enzyme | | 10µl α-amylase enzyme |
| | | | 90µl sterile water |

Incubation and plating as described in the general section.

### 5. Trypsin.

Preparation buffer: 50mM Tris pH8.0 and 10mM CaCl₂
(93ml sterile water + 5ml 1M Tris pH8.0 + 2ml 0.5M CaCl₂)
Preparation 10mg/ml solution of trypsin enzyme in buffer
Enzyme analysis in triplicate of 2 concentrations of trypsin (*High* and *Low*)

| | | | |
|---|---|---|---|
| *High* | 800µl buffer | *Low* | 800µl buffer |
| | 100µl spore suspension | | 100µl spore suspension |
| | 100µl trypsin enzyme | | 10µl trypsin enzyme |
| | | | 90µl sterile water |

Incubation and plating as described in the general section.

### 6. α-chvmotrypsin.

The same buffer is applied as in the trypsin experiment (50mM Tris pH8.0 and 10mM CaCl₂)
Preparation 10mg/ml solution of α-chymotrypsin enzyme in buffer
Enzyme analysis in triplicate of 2 concentrations of α-chymotrypsin (*High* and *Low*)

| | | | |
|---|---|---|---|
| *High* | 800µl buffer | *Low* | 800µl buffer |
| | 100µl spore suspension | | 100µl spore suspension |
| | 100µl α-chymotrypsin | | 10µl α-chymotrypsin |
| | | | 90µl sterile water |

Incubation and plating as described in the general section.

### 7. Glucose oxidase.

Preparation 100mM acetate buffer with pH5.1
(0.41 g Na-acetate in 50ml sterile water, pH is adjusted to 5.1 with HCl)
Preparation 10mg/ml solution of glucose oxidase enzyme in buffer Enzyme analysis in triplicate
Two concentrations of glucose oxidase is used (*High* and *Low*), both plus and minus glucose

| | | | |
|---|---|---|---|
| *High-P* | 500µl buffer | *Low-P* | 500µl buffer |
| | 100µl spore suspension | | 100µl spore suspension |
| | 100µl glucose oxidase | | 10µl glucose oxidase |
| | 50µl 20% glucose | | 50µl 20% glucose |
| | 250µl sterile water | | 340µl sterile water |
| *High-M* | 500µl buffer | *Low-M* | 500µl buffer |
| | 100µl spore suspension | | 100µl spore suspension |
| | 100µl glucose oxidase | | 10µl glucose oxidase |
| | 300µl sterile water | | 390µl sterile water |

Incubation and plating as described in the general section.
(Double experiment and utilization of 24 plates)

### 8. Phospholipase A₂.

Preparation buffer: 10mM Tris pH8.0, 20mM CaCl₂ and 150mM NaCl
(92ml sterile water + 1 ml 1M Tris pH8.0 + 4ml 0.5M CaCl₂ + 3ml 5M NaCl)
Preparation 10mg/ml solution of phospholipase A₂ enzyme in buffer Enzyme analysis in triplicate for 2 concentrations of phospholipase A₂ *(High* and *Low*)

| | | | |
|---|---|---|---|
| *High* | 800µl buffer | *Low* | 800µl buffer |
| | 100µl spore suspension | | 100µl spore suspension |
| | 100µl phospholipase A2 | | 10µl phospholipase A2 |
| | | | 90µl sterile water |

Incubation and plating as described in the general section.

### 9. Direct plating.

Dilution and plating of stock solution the same day.
Measurement is carried out in triplicate =3 identical samples
Stock solution is diluted first 100x (times 10x dilution in sterile water)
Then it is diluted 20x (50µl resuspension + 900µl sterile water)
(Total dilution = 2000x)
100µl and 50µl is plated on Nutrient Broth agar plates
(Altogether 6 plates)
Incubation of plates at 30°C and count of colonies after o/n incubation

### Plate count results (cfu):

### 1. Lysozyme

| | | | | | **Average** |
|---|---|---|---|---|---|
| Lysozyme High | | #1 | #2 | #3 | |
| | 200µl | 175 | 126 | 199 | **167** |
| | 100µl | 80 | 83 | 118 | **94** |
| | | | | | |

| Lysozyme Low | | #1 | #2 | #3 | |
|---|---|---|---|---|---|
| | 200µl | 143 | 166 | 163 | **157** |
| | 100µl | 97 | 139 | 91 | **109** |
| | | | | | |
| Calculated total average (100 µl sample): | | | | **91** | |

### 2. Keratanase

| Keratanase High | | #1 | #2 | #3 | |
|---|---|---|---|---|---|
| | 200µl | 138 | 142 | 137 | **139** |
| | 100µl | 70 | 86 | 69 | **75** |
| | | | | | |

| Keratanase Low | | #1 | #2 | #3 | |
|---|---|---|---|---|---|
| | 200µl | 181 | * | 143 | **162** |
| | 100µl | 74 | 88 | 66 | **76** |
| *: *Plate not included due to contamination* | | | | | |
| | | | | | |
| Calculated total average (100 µl sample): | | | | **75** | |

### 3. Protease

| Protease High | | #1 | #2 | #3 | |
|---|---|---|---|---|---|
| | 200µl | 186 | 183 | 223 | **197** |
| | 100µl | 60 | 77 | 124 | **87** |
| | | | | | |

| Protease Low | | #1 | #2 | #3 | |
|---|---|---|---|---|---|
| | 200µl | 159 | 5* | 190 | **175** |
| | 100µl | 86 | 10* | 97 | **92** |
| *: *Data not included in analysis (unknown cause of deviation)* | | | | | |
| Calculated total average (100 µl sample): | | | | **91** | |

### 4. Alfa-amylase

| | | | | | **Average** |
|---|---|---|---|---|---|
| | | | | | |

| Alfa-amylase High | | #1 | #2 | #3 | |
|---|---|---|---|---|---|
| | 200µl | 239 | 276 | 277 | **264** |
| | 100µl | 131 | 165 | 177 | **158** |
| | | | | | |

| Alfa-amylase Low | | #1 | #2 | #3 | |
|---|---|---|---|---|---|
| | 200µl | 254 | 265 | 263 | **261** |
| | 100µl | 127 | 164 | 179 | **157** |
| | | | | | |
| Calculated total average (100 µl sample): | | | | **144** | |

### 5. Trypsin

| Trypsin High | | #1 | #2 | #3 | |
|---|---|---|---|---|---|
| | 200µl | 167 | 168 | 171 | **169** |
| | 100µl | 83 | 92 | 85 | **87** |
| | | | | | |

| Trypsin Low | | #1 | #2 | #3 | |
|---|---|---|---|---|---|
| | 200µl | 160 | 160 | 173 | **164** |
| | 100µl | 102 | 93 | 73 | **89** |
| | | | | | |
| Calculated total average (100 µl sample): | | | | **86** | |

### 6. Alfa-chymotrypsin

| Alfa-chymotrypsin High | | #1 | #2 | #3 | |
|---|---|---|---|---|---|
| | 200µl | 229 | 232 | 263 | **241** |
| | 100µl | 130 | 127 | 158 | **138** |
| | | | | | |

| Alfa-chymotrypsin Low | | #1 | #2 | #3 | |
|---|---|---|---|---|---|
| | 200µl | 208 | 224 | 229 | **220** |
| | 100µl | 110 | 123 | 149 | **127** |
| | | | | | |
| Apparently identical High and Low values. Calculated total average (100 µl sample): | | | | **124** | |

### 7. Glucose oxidase (+ glucose)

| Glucose oxidase High P | | #1 | #2 | #3 | |
|---|---|---|---|---|---|
| | 200µl | 0 | 0 | 0 | **0** |
| | 100µl | 0 | 0 | 0 | **0** |
| | | | | | **Average** |

| Glucose oxidase Low P | | #1 | #2 | #3 | |
|---|---|---|---|---|---|
| | 200µl | 0 | 0 | 1 | **0** |
| | 100µl | 0 | 0 | 0 | **0** |
| | | | | | |
| Calculated total average (100 µl sample): | | | | **0** | |

### 7. Glucose oxidase (without glucose)

| Glucose oxidase High M | | #1 | #2 | #3 | |
|---|---|---|---|---|---|
| | 200µl | 195 | 196 | 242 | **211** |
| | 100µl | 136 | 102 | 104 | **114** |
| | | | | | |

| Glucose oxidase Low M | | #1 | #2 | #3 | |
|---|---|---|---|---|---|
| | 200µl | 10 | 14 | 28 | **17** |
| | 100µl | 2 | 0 | 13 | **5** |
| | | | | | |
| Calculated total average High (100 µl sample) | | | | **110** | |
| Calculated total average Low (100 µl sample) | | | | **7** | |

### 8. Phospholipase A2

| Phospholipase A2 High | | #1 | #2 | #3 | |
|---|---|---|---|---|---|
| | 200µl | 233 | 200 | 237 | **223** |
| | 100µl | 121 | 123 | 117 | **120** |
| | | | | | |

| Phospholipase A2 Low | | #1 | #2 | #3 | |
|---|---|---|---|---|---|
| | 200µl | 247 | 237 | 294 | **259** |
| | 100µl | 149 | 107 | 132 | **129** |
| | | | | | |
| Calculated total average (100 µl sample): | | | | **121** | |

### 9. Direct plating

| Direct plating | | #1 | #2 | #3 | |
|---|---|---|---|---|---|
| 200µl | | 392 | 380 | 392 | **388** |
| 100µl | | 201 | 207 | 182 | **197** |
| | | | | | |
| Calculated total average (100 µl sample): | | | | **195** | |

### EXAMPLE 6

The aim of this study is to investigate how *Bacillus atrophaeus* spores respond to a combined treatment using both enzymes and ozone. The study was a follow-up to a study only using enzyme treatment. Bacillus atrophaeus spores are highly resistant to heat and chemicals and their use are officially recognized for sterilization procedure certification.
Novel sterilization procedures are needed for medical equipment containing sensitive materials that do not tolerate high temperatures and hence cannot be sterilized by use of heat. Here 7 different commercially available enzymes are investigated for a possible use prior to an ozone treatment.

### Materials and Experimental procedure

### Materials:

*Bacillus atrophaeus* spores, enzymes and medium: see Example 5

### Procedure:

Spores and enzymes were incubated in buffer solutions as described in Appendix. The mixtures were incubated at 37°C for 2 hours and then filtered as described in Appendix. Filters were cut into halves, and one half treated with ozone for 30 min as described in Appendix. Both halves were finally placed onto a Nutrient Agar plate and incubated at 30°C.
The number of Bacillus atrophaeus colonies on the two filter halves was compared visually. Work was divided into 3 experiments (A, B and C). The first experiment (A) employed glucose oxidase as the enzyme tested, while the remaining 6 enzymes were tested in a second experiment (B). Finally, a third experiment (C) was performed using two enzymes, glucose oxidase and phospholipase A2, and using direct plating instead of filtering as described in the Appendix.

### Results:

Direct plating of spores on Nutrient Agar plates showed low and decreasing colony counts as a function of incubation times (results for experiment 3 described in Appendix). It is suggested that potential differences in spore sensitivity for ozone treatment on agar plates compared to filters are due to the moist germinating conditions found on the filter. Ozone treatment was found to reduce colony counts irrespective of the enzyme concentration used.

### Appendix

### Materials and Methods:

### Preparation of spore suspension:

A sterile tube is added 10ml buffer
Addition of 5pcs of *Bacillus atrophaeus* discs
Whirl mix for 2min for liberation and suspension of spores
Concentration: 5x10⁵ spores/ml
Each stock solution is used for an entire test series.

### Experiment A:

Experiment with glucose oxidase
Preparation of 100mM acetate buffer with pH 5:1
(0.41 g Na-acetate in 50 ml sterile water, pH is adjusted to 5.1 with HCl)

Preparation of 10mg/ml solution of glucose oxidase enzyme in buffer
Preparation of spore suspension in buffer (1 pcs)
Use 1.5-ml Eppendorf tube, two concentrations of glucose oxidase
Four tubes of each type is prepared

| | |
|---|---|
| *High* | 350 µl buffer |
| | 500 µl spore suspension |
| | 100 µl glucose oxidase |
| | 50 µl 20% glucose |
| | |
| *Low* | 440 µl buffer |
| | 500 µl spore suspension |
| | 10 µl glucose oxidase |
| | 50 µl 20% glucose |

### Filtration:

The tubes are incubated for 2hours before filtration
The contents of the tubes (1 ml) is mixed with 10ml sterile water
Filtration (disposable filter on 500-ml Erlenmeyer filtering flask)
Wash of filters with 10ml sterile water
With a sterile forceps the filter is transferred to a Petri dish (labelled with sample-ID)
With a sterile pair of scissors the filter is cut in two halves
One half is placed directly on a Nutrient Agar Plate (labelled with sample-ID)
The other half is treated with ozone (remains in the labelled Petri dish)

### Ozone treatment:

Open Petri dishes with filter halves is placed in the OBOX (see Example 4) and treated for 30min at 40-60 ppm ozone.

### Incubation:

The ozone-treated halves are placed on the Nutrient Agar plates with their respective counterparts using sterile forceps, and while taking care that no air bubbles were trapped between filter and agar. Plates are incubated o/n at 30°C

### Experiment B:

Experiment with six different enzymes

### 1. Lysozyme:

Preparation 10mg/ml solution of lysozyme in STET buffer
(STET buffer: 8% sucrose, 5% TritonX-100, 50mM Tris pH8.0, 50mM EDTA)

### 2. Protease:

Preparation 10mM Tris pH8.0
(99ml sterile water + 1 ml 1M Tris pH8.0)
Enzyme is drawn directly from tube (liquid product)

### 3. α-amylase

Preparation 50mM Tris pH 8.0
(95ml sterile water + 5ml 1M Tris pH8.0)
Preparation 10mg/ml solution of alfa-amylase enzyme in Tris buffer

### 4. Trypsin.

Preparation buffer: 50mM Tris pH8.0 and 10mM CaCl₂
(93ml sterile water + 5ml 1M Tris pH8.0 + 2ml 0.5M CaCl₂)
Preparation 10mg/ml solution of trypsin enzyme in buffer

### 5. α-chymotrypsin.

The same buffer is applied as in the trypsin experiment (50mM Tris pH8.0 and 10mM CaCl₂)
Preparation 10mg/ml solution of alfa-chymotrypsin enzyme in buffer

### 6. Phospholipase A₂.

Preparation buffer: 10mM Tris pH 8.0, 20mM CaCl₂ and 150mM NaCl
(92ml sterile water + 1ml 1M Tris pH8.0 + 4ml 0.5M CaCl₂ + 3ml 5M NaCl)
Preparation 10mg/ml solution of phospholipase A₂ enzyme in buffer

### Preparation of spore suspension:

A sterile tube is added 10ml buffer
Addition of 5pcs of *Bacillus atrophaeus* discs
Whirl mix for 2min for liberation and suspension of spores
Concentration: 5x10⁵ spores/ml

### Procedure:

Use 1.5-ml eppendorf tubes.
All experiments are carried out in duplicates, as two samples are prepared from each of the following solutions.
Since only six samples can be ozone-treated at a time, the experiments are started with the preparation of tubes for samples 1-3. With a delay of app. one hour, the experiments and preparation of tubes for samples 4-6 is started.

### Preparation mix at start t = 0 h

(1) 400 µl buffer
   500 µl spore suspension
   100 µl lysozyme solution
(2) 400 µl buffer
   500 µl spore suspension
   100 µl protease enzyme
(3) 400 µl buffer
   500 µl spore suspension
   100 µl α-amylase solution

### Preparation mix at start t = ∼1 hour

(4) 400 µl buffer
   500 µl spore suspension
   100 µl trypsin solution
(5) 400 ml buffer
   500 µl spore suspension
   100 µl α-chymotrypsin solution
(6) 400 µl buffer
   500 µl spore suspension
   100 µl phospholipase A₂ enzyme solution

### Incubation:

All tubes are incubated at 37°C for 2hours with agitation

### Filtration-1: (the first six samples)

Contents of tubes (1 ml) are mixed with 10ml sterile water
Filtration (disposable filter on 500-ml Erlenmeyer filtering flask)
Wash of filters with 10ml sterile water
With a sterile forceps the filter is transferred to a Petri dish (labelled with sample-ID)
With a sterile pair of scissors the filter is cut in two halves
One half is placed directly on a Nutrient Agar Plate (labelled with sample-ID) The other half is treated with ozone (remains in the labelled Petri dish)

### Ozone treatment-1:

Open Petri dishes with filter halves is placed in the OBOX (see Example 4) and treated for 30min at 40-60 ppm ozone.

### Incubation:

The ozone-treated halves are placed on the Nutrient Agar plates with their respective counterparts using sterile forceps, and while taking care that no air bubbles were trapped between filter and agar. Plates are incubated o/n at 30°C

### Filtration-2: (app. 1 hour later, the next six samples)

Procedure as described above.

### Ozone treatment-2 + incubation:

Procedure as described above.

### Experiment C:

Further experiment with Glucose oxidase and Phospholipase A₂

### Preparation of spore suspension:

A sterile tube is added 10ml water
Addition of 5pcs of *Bacillus atrophaeus* discs
Whirl mix for 2min for liberation and suspension of spores
Concentration: 5x10⁵ spores/ml

### Procedure:

Use 1.5-ml eppendorf tubes (12 tubes in total)
Experiments are carried out with high and low amounts of enzyme. Three tubes are prepared of each of the following solutions.
Experiments are carried out with three different incubation times (enzyme treatment in 1, 2 and 3hours). Altogether six samples for ozone treatment for each incubation time.

### Glucose oxidase

Preparation 100mM acetate buffer with pH5.1
(0,41 g Na-acetate in 50ml sterile water, pH is adjusted to 5.1 with HCl)

| | |
|---|---|
| *High* | 650 µl buffer |
| | 200 µl spore suspension |
| | 100 µl glucose oxidase solution |
| | 50 µl 20% glucose |
| | |
| *Low* | 650 µl buffer |
| | 200 µl spore suspension |
| | 90 µl sterile water |
| | 10 µl glucose oxidase solution |
| | 50 µl 20% glucose |

### Phospholipase A₂

Preparation buffer: 10mM Tris pH8.0, 20mM CaCl₂ and 150mM NaCl
(92 ml sterile water + 1 ml 1M Tris pH8.0 + 4ml 0.5M CaCl₂ + 3ml 5M NaCl)
Preparation 10 mg/ml solution of phospholipase A₂ enzyme in buffer

| | |
|---|---|
| *High* | 700 µl buffer |
| | 200 µl spore suspension |
| | 100 µl phospholipase A₂ solution |
| | |
| *Low* | 700 µl buffer |
| | 200 µl spore suspension |
| | 90 µl sterile water |
| | 10 µl phospholipase A₂ solution |

### Enzyme treatment:

The samples are incubated at 37°C for 1, 2 or3h with agitation as previously described.

### Plating:

Preparation 100x dilution (carried out in two rounds via 100µl sample + 900µl sterile water)
100µl dilution is plated on two Nutrient Agar plates
One plate is treated with ozone
The other plate functions as control (no ozone treatment)
(Altogether 12 plates are used per hour, of which six are treated with ozone)

### Ozone treatment and incubation:

Ozone treatment and incubations were performed as previously described.

**Table 3. Comparison of glucose oxidase and phospholipase A2 treatments +/- ozone.**

| **Glucose oxidase** | | | | **Phospholipase A2** | | |
|---|---|---|---|---|---|---|
| | | | | | | |
| **1 time** | | | | **1 time** | | |
| | - ozon | + ozon | | | - ozon | + ozon |
| High | 81 | 13 | | High | 76 | 33 |
| Low | 70 | 0 | | Low | 50 | 0 |
| | | | | | | |

| **2 timer** | | | | **2 timer** | | |
|---|---|---|---|---|---|---|
| | - ozon | + ozon | | | - ozon | + ozon |
| High | 21 | 0 | | High | 64 | 15 |
| Low | 4 | 0 | | Low | 44 | 9 |
| | | | | | | |

| **3 timer** | | | | | | |
|---|---|---|---|---|---|---|
| | - ozon | + ozon | | | | |
| High | 0 | 0 | | | | |
| Low | 1 | 0 | | | | |

### EXAMPLE 7

The aim of this experiment was to investigate if an antimicrobial activity of a solution of a commercially available household dish-washing powder can be destroyed by heating, and if the antimicrobial activity can be restored by addition of enzymes and ozone treatment.

### Materials and Methods:

**(1)** 25g Neophos Powder ("NeoP"; i.e. a commercially available dish-washing powder, containing 15-30% Phosphates, < 5% bleaches with oxygen, nonionic tensides, perfume (limonene), enzymes (Protease, Amylase); trade name neophos may change to "finish"; Reckitt Benckiser (Scandinavia) A/S, DK-2880 Bagsværd, Denmark; www.neophos.dk; Batch nr. L7 103 M DK; recommended dosage: 40g (36mL) for normal use in a dish washer) is dissolved in 250mL ∼70°C water and a "Raven disk" (stainless steel disc with ∼1-2 x 10⁶ *Bacillus atrophaeus* spores (log 6, cat# 1-6100ST, Raven Biological Laboratories)) is incubated in 2mL of this solution at 57°C for ∼1h (in duplicate) without sonication or vortexing. A dilution series is prepared from 0.5mL of the incubated solution in TSB with Phenol Red (10⁻¹, 10⁻², 10⁻³, 10⁻⁴, 10⁻⁵ and 10⁻⁶). 250µL of the original incubated solution is filtered, while 9mL of the dilutions 10⁻², 10⁻³, 10⁻⁴ and 10⁻⁵ are filtered; for technical and practical reasons 12.5mL sterile water is added to the sample. The filter is rinsed by filtering 2x 12.5mL sterile water and the unit is disassembled aseptically. Filters are cut in halves, and one half is exposed to 40-60ppm ozone in an OBOX (see Example 4 for further details), while the other half remains untreated. The filters are transferred to TSA plates and incubated overnight at ∼35°C as described earlier.
**(2)** In a parallel experiment, 50mL Neophos powder (NeoP) solution from above is heat-inactivated at 80°C for 75min before a "Raven disc" is incubated in 2mL of this solution at 57°C for 45min (in duplicate), again without sonication or vortexing. Dilution series, filtering and +/- ozone treatment as well as transfer to TSA plates and incubation are performed essentially as described above.
**(3)** To 2 mL of the heat-inactivated NeoP solution from (2), either 10µL alpha-amylase (1.0U), 100µL protease (0.5U), or a combination of protease (0.5U) and alpha-amylase( 1.0U) is added.
   α- amylase (*Bacillus subtilis*) was purchased from FLUKA (*10070 FLUKA,* lot&filling code: 1323787 31707211, CAS: 9000-90-2, EC: 3.2.1.1, Powder (55U/mg)); working stock: 2mg/mL in sterile water.
   Protease (Bacillus sp.), was purchased from Sigma (*P5985 Sigma* (50mL), batch: 016K1071, CAS: 9014-01-1; EC: 232-752-2; Liquid solution (∼16U/mg); working stock: 3mL enzyme solution + 7mL sterile water
   After enzyme addition, one "Raven disc" is incubated per assay in these solutions at 57°C for 1 hour (in duplicate), again without sonication or vortexing. Also dilution series, filtering and +/- ozone treatment as well as transfer to TSA plates and incubation are performed essentially as described above.

### Results

**(1)** No growth was detected in the original after 45min treatment with active Neophos powder, irrespective of ozone treatment or not.
**(2)** *Table 4. Dilution series and cfu counts of heat inactivated NeoP solution.*

| | | | |
|---|---|---|---|
| Dilution | 10⁻⁵: | 10⁻⁴: | 10⁻³: |
| Colonies | 19 cfu | -200 cfu | ∼2.000 cfu |

The results of cfu counts (average of to repetitions) presented in Table 4 show that the inactivated solution was not able to kill spores, and showed virtually no antimicrobial activity. The numbers correspond to 2 x 10⁶ cfu in the solution, which is within the range stated by the disc supplier (1-4 x 10⁶ cfu).
**(3)** *Table 5. Cfu counts of active, inactivated NeoP solution, restored NeoP solution (+* α*-amylase, protease and both* α*-amylase + protease) and comparison +*/*- ozone treatment.*

| | **Active NeoP** | **Inactivated NeoP** | **+ α-amylase** | **+ protease** | **+ α-amylase + protease** |
|---|---|---|---|---|---|
| **- O₃** | 0 cfu | 2 x 10⁶ cfu | 5.2 x 10⁵ cfu | 8.3 x 10⁴ cfu | 1.5 x 10⁴ cfu |
| **+ O₃** | 0 cfu | 2 x 10⁶ cfu | 1.1 x 10⁵ cfu | 2.1 x10³ cfu | <10 cfu |

The results obtained from cfu counts (average of to repetitions) derived from the experimental evidence presented in Figure 6, as well as Table 5 reveal the following:
A concentrated NeoP solution (-50 x more concentrated than in a standard dishwasher) is efficiently killing spores. This antimicrobial activity is lost upon heat treatment. Addition of α-amylase can partially restore the lost antimicrobial activity of heat treated NeoP solution. Addition of protease restores more antimicrobial activity than α-amylase. Addition of α-amylase and protease restores antimicrobial activity even more.
A consecutive treatment with ozone increases the antimicrobial activity upon addition of α-amylase or protease. Surprisingly and unexpectedly, this increase in antimicrobial activity upon ozone treatment was much stronger for the samples, where both α-amytase and protease were added to the inactivated NeoP solution. This combination provided a 5-6 log reduction in cfu.

## Claims

1. A method for sterilization or disinfection, comprising the steps of:
(a) contacting one or more item or part of said item with a water-based fluid containing at least one enzyme;
(b) a rinsing step; and
(c) contacting said one or more item or part of said item with a gas having oxidative properties;
wherein step (a) precedes step (b), and step (b) precedes step (c); wherein step (b) comprises removal of water based fluid and flushing with processed gas, whereby a substantially water-free environment is provided which does not contain visible traces of humidity; and wherein said gas having oxidative properties is selected from the group consisting of ozone, oxygen, ethylene oxide, and hydrogen peroxide.

2. A method according to claim 1, wherein said step (a) further comprises an ultrasonic treatment; and/or wherein said water based fluid containing at least one enzyme is provided as cold steam.

3. A method according to claim 1 or 2, wherein said processed gas is dry or dried air, ambient air with a lower humidity than ambient air, ambient air, or nitrogen, optionally filtered or sterile filtered.

4. A method according to any one of the preceding claims, wherein said rinsing step (b) further comprises contacting said one or more item or part of said item with processed water, wherein said processed water is demineralized water, tap water, or sterile water.

5. A method according to any one of the preceding claims, wherein said gas having oxidative properties is ozone; and/or wherein said gas with oxidative properties in said step (c) comprises ozone in the range of 1 to 10000 ppm, 5 to 100 ppm, 10 to 60 ppm, 40-60 ppm, 30-50 ppm, or 15-30 ppm.

6. A method according to any one of the preceding claims, wherein said enzyme is selected from the group consisting of one or more of cell wall-modifying or -degrading enzyme, protein-modifying or -degrading enzyme, and/or fat-modifying or -degrading enzyme.

7. A method according to claim 6, wherein said enzyme is selected from the group consisting of one or more cellulase, chitinase, amylase, protease, and/or lipase; and/or wherein said enzyme is capable of impairing bacterial spores sufficiently to render said bacterial spores susceptible for sterilization by ozone gas.

8. A method according to any one of the preceding claims, wherein said steps (a) and (c) are performed at a maximum temperature not exceeding 100°C, 70°C, 50°C, 37°C, 25°C, 20°C or ambient temperature; and/or
wherein said step (a) is performed at a pH in the range of pH 2 to 12, 4 to 10, 6 to 8, or 6.8 to 7.2; and/or
wherein the pressure applied is in the range of 1 to 300 kPa, 10 to 200 kPa, 50 to 150 kPa, or 80 to 120kPa during said steps (a) and/or (c).

9. A method according to any one of the preceding claims, wherein said steps (a) and (c) are performed in a total time not exceeding 60 min, 30 min, 15 min, or 5 min.

10. A method according to claim 9, wherein the total time for said step (a) does not exceed 30 min, 10 min, 5 min, or 2 min.

11. A method according to claim 9 or 10, wherein the total time for said step (c) does not exceed 20 min, 10 min, 5 min, or 1 min.

12. A method according to any of the preceding claims, wherein said one or more item or part of said one or more item is selected from laboratory items, medical items, dental items, military items, biological items, and/or food processing-related items.

13. A method according to claim 12, wherein said one or more item or part of said one or more item is sensitive to or impaired by temperature, pH, positive or negative pressure, radiation and/or oxidation, if subjected to other forms of sterilization and/or disinfection.

14. A method according to claim 12 or 13, wherein said one or more item or part of said one or more item is a disposable item, i.e. an item or part of an item not intended for re-use.

15. A method according to claim 13, wherein said one or more item or part of said one or more item is an endoscope.

## Patentansprüche

1. Verfahren zur Sterilisation oder Desinfektion, umfassend die folgenden Schritte:
(a) das Inkontaktbringen einer oder mehrerer Einheiten oder eines Teils der Einheit mit einem wasserbasierten, mindestens ein Enzym enthaltenden Fluid;
(b) einen Spülschritt; und
(c) das Inkontaktbringen der einen oder mehreren Einheiten oder des Teils der Einheit mit einem Gas mit oxidativen Eigenschaften;
wobei der Schritt (a) dem Schritt (b) vorausgeht, und der Schritt (b) dem Schritt (c) vorausgeht, wobei der Schritt (b) ein Entfernen wasserbasierten Fluids und ein Durchspülen mit behandeltem Gas umfasst, wodurch eine im Wesentlichen wasserfreie Umgebung vorgesehen wird, welche keine erkennbaren Spuren von Feuchtigkeit aufweist; und wobei das Gas mit oxidativen Eigenschaften aus der Gruppe bestehend aus Ozon, Oxygen, Ethylenoxid und Hydrogenperoxid ausgewählt ist.

2. Verfahren nach Anspruch 1, wobei der Schritt (a) ferner eine Ultraschallbehandlung umfasst; und/oder wobei das mindestens ein Enzym enthaltende wasserbasierte Fluid als Kaltdampf vorgesehen ist.

3. Verfahren nach Anspruch 1 oder 2, wobei das behandelte Gas trockene oder getrocknete Luft, Raumluft mit einer niedrigeren Feuchtigkeit als Raumluft, Raumluft oder Stickstoff, wahlweise gefiltert oder sterilgefiltert, darstellt.

4. Verfahren nach einem der vorgehenden Ansprüche, wobei der Spülschritt (b) ferner das Inkontaktbringen der einen oder mehreren Einheiten oder des Teils der Einheit mit behandeltem Wasser umfasst, wobei das behandelte Wasser demineralisiertes Wasser, Leitungswasser oder steriles Wasser ist.

5. Verfahren nach einem der vorgehenden Ansprüche, wobei das Gas mit oxidativen Eigenschaften Ozon ist; und/oder wobei das Gas mit oxidativen Eigenschaften in dem Schritt (c) Ozon im Bereich von 1 bis 10.000 ppm, 5 bis 100 ppm, 10 bis 60 ppm, 40-60 ppm, 30-50 ppm oder 15-30 ppm umfasst.

6. Verfahren nach einem der vorgehenden Ansprüche, wobei das Enzym ausgewählt ist aus der Gruppe bestehend aus einem oder mehreren von Zellenwand-modifizierendem oder -abbauendem Enzym, Protein-modifizierendem oder -abbauendem Enzym und/oder Fett-modifizierendem oder -abbauendem Enzym.

7. Verfahren nach Anspruch 6, wobei das Enzym ausgewählt ist aus der Gruppe bestehend aus einem oder mehreren von Cellulase, Chitinase, Amylase, Protease und/oder Lipase; und/oder wobei das Enzym dazu im Stande ist, bakterielle Sporen ausreichend zu beeinträchtigen, um die bakteriellen Sporen gegenüber Sterilisation durch Ozongas empfindlich zu machen.

8. Verfahren nach einem der vorgehenden Ansprüche, wobei die Schritte (a) und (c) bei einer Maximaltemperatur durchgeführt werden, die eine Temperatur von 100 °C, 70 °C, 50 °C, 37 °C, 25 °C, 20 °C oder Raumtemperatur nicht überscheitet; und/oder
wobei der Schritt (a) bei einem pH-Wert im Bereich von pH 2 bis 12, 4 bis 10, 6 bis 8 oder 6,8 bis 7,2 durchgeführt wird; und/oder wobei der ausgeübte Druck während der Schritte (a) und/oder (c) im Bereich von 1 bis 300 kPa, 10 bis 200 kPa, 50 bis 150 kPa oder 80 bis 120 kPa liegt.

9. Verfahren nach einem der vorgehenden Ansprüche, wobei die Schritte (a) und (c) über eine Gesamtdauer durchgeführt werden, die einen Zeitraum von 60 min, 30 min, 15 min oder 5 min nicht überschreitet.

10. Verfahren nach Anspruch 9, wobei die Gesamtdauer des Schrittes (a) nicht 30 min, 10 min, 5 min oder 2 min überschreitet.

11. Verfahren nach Anspruch 9 oder 10, wobei die Gesamtdauer des Schrittes (c) nicht 20 min, 10 min, 5 min oder 1 min überschreitet.

12. Verfahren nach einem der vorgehenden Ansprüche, wobei die eine oder mehreren Einheiten oder der Teil der einen oder der mehreren Einheiten aus Laboreinheiten, medizinischen Einheiten, dentalen Einheiten, militärischen Einheiten, biologischen Einheiten und/oder lebensmittelverarbeitungsbezogenen Einheiten ausgewählt ist.

13. Verfahren nach Anspruch 12, wobei die eine oder mehreren Einheiten oder der Teil der einen oder der mehreren Einheiten gegenüber Temperatur, pH-Wert, positivem oder negativem Druck, Strahlung und/oder Oxidation empfindlich sind oder durch diese Faktoren beeinträchtigt werden, wenn sie anderen Formen von Sterilisation und/oder Desinfektion ausgesetzt werden.

14. Verfahren nach Anspruch 12 oder 13, wobei die eine oder mehreren Einheiten oder der Teil der einen oder mehreren Einheiten eine Einweg-Einheit ist, d.h. eine Einheit oder ein Teil einer Einheit, die für Wiederverwendung nicht bestimmt ist.

15. Verfahren nach Anspruch 13, wobei die eine oder mehreren Einheiten oder der Teil der einen oder mehreren Einheiten ein Endoskop ist.

## Revendications

1. Procédé pour la stérilisation ou désinfection comprenant les étapes suivantes :
(a) amener en contact un ou plusieurs éléments ou partie dudit élément avec un fluide sur base d'eau contenant ou moins une enzyme ;
(b) une étape de rinçage ; et
(c) amener en contact ledit un ou lesdits plusieurs éléments ou partie dudit élément avec un gaz ayant des propriétés oxydatives ;
dans lequel l'étape (a) précède l'étape (b), et l'étape (b) précède l'étape (c) ; dans lequel l'étape (b) comprend l'évacuation de fluide sur base d'eau et le rinçage avec du gaz traité, ainsi fournissant un milieu essentiellement sans eau qui ne contient pas de traces visibles d'humidité ; et
dans lequel ledit gaz ayant des propriétés oxydatives est choisi parmi le groupe consistant d'ozone, d'oxygène, d'oxyde d'éthylène et de peroxyde d'hydrogène.

2. Procédé selon la revendication 1, dans lequel ladite étape (a) comprend en outre un traitement ultrasonique ; et/ou dans lequel ledit fluide sur base d'eau contenant au moins une enzyme est pourvu comme vapeur froid.

3. Procédé selon les revendications 1 ou 2, dans lequel ledit gaz traité est de l'air sèche ou séché, de l'air ambiante avec une humidité plus basse que celle de l'air ambiante, de l'air ambiante, ou nitrogène, éventuellement filtrée ou filtrée sous conditions stériles.

4. Procédé selon l'une quelconque des revendications précédentes, dans lequel ladite étape de rinçage (b) comprend en outre d'amener en contact ledit un ou lesdits plusieurs éléments ou partie dudit élément avec de l'eau traitée, dans lequel ladite eau traitée est de l'eau déminéralisée, de l'eau de canalisation, ou bien de l'eau stérile.

5. Procédé selon l'une quelconque des revendications précédentes, dans lequel ledit gaz ayant des propriétés oxydatives est de l'ozone ; et/ou dans lequel ledit gaz ayant des propriétés oxydatives, en étape c), comprend de l'ozone dans la gamme de 1 à 10.000 ppm, de 5 à 100 ppm, de 10 à 60 ppm, 40 - 60 ppm, 30 - 50 ppm, ou bien 15 - 30 ppm.

6. Procédé selon l'une quelconque des revendications précédentes, dans lequel ladite enzyme est choisie parmi le groupe consistant d'un ou plusieurs d'enzymes modifiant ou dégradant la paroi cellulaire, d'enzymes modifiant ou dégradant la protéine, et/ou d'enzymes modifiant ou dégradant de la grasse.

7. Procédé selon la revendication 6, dans lequel ladite enzyme est choisie parmi le groupe consistant d'une ou plusieurs de cellulase, chitinase, amylase, protéase, et/ou lipase ; et/ou dans lequel ladite enzyme est capable de détériorer suffisamment les spores bactériennes pour rendre lesdites spores bactériennes susceptibles pour la stérilisation par gaz d'ozone.

8. Procédé selon l'une quelconque des revendications précédentes, dans lequel les étapes (a) et (c) sont exécutées à une température maximale ne dépassant pas 100 °C, 70 °C, 50 °C, 37 °C, 25 °C, 20 °C ou bien la température ambiante ; et/ou
dans lequel ladite étape (3) est exécutée à une pH dans la gamme de pH 2 à 12, 4 à 10, 6 à 8, ou 6, 8 à 7,2; and/or
dans lequel la pression appliquée est dans la gamme de 1 à 300 kPa, 10 à 200 kPa, 50 à 150 kPa, ou 80 à 120 kPa lors desdits étapes (a) et/ou (c).

9. Procédé selon l'une quelconque des revendications précédentes, dans lequel les étapes (a) et (c) sont exécutés dans un temps total ne dépassant pas 60 minutes, 30 minutes, 15 minutes, ou 5 minutes.

10. Procédé selon la revendication 9, dans lequel le temps total pour ladite étape (a) ne dépasse pas 30 minutes, 10 minutes, 5 minutes, ou 2 minutes.

11. Procédé selon les revendications 9 ou 10, dans lequel le temps total pour ladite étape (a) ne dépasse pas 20 minutes, 10 minutes, 5 minutes, ou 1 minute.

12. Procédé selon l'une quelconque des revendications précédentes, dans lequel ledit un ou lesdits plusieurs éléments est choisi parmi d'éléments à usage laboratoire, d'éléments à usage médical, d'éléments à usage dental, d'éléments d'usage militaire, d'éléments d'usage biologique, et/ou d'éléments relatifs au traitement des produits alimentaires.

13. Procédé selon la revendication 12, dans lequel ledit un ou lesdits plusieurs éléments ou partie dudit un ou desdits plusieurs éléments sont sensibles à ou détériorés par la température, le pH, la pression positive ou négative, la radiation et/ou l'oxydation dans le cas où ils sont soumis aux autres types de stérilisation et/ou désinfection.

14. Procédé selon les revendications 12 ou 13, dans lequel ledit un ou lesdits plusieurs éléments ou partie dudit un ou desdits plusieurs éléments est un élément jetable, c'est-à-dire un élément ou une partie d'un élément pas destiné pour la réutilisation.

15. Procédé selon la revendication 13, dans lequel ledit un ou lesdits plusieurs éléments ou partie dudit un ou desdits plusieurs éléments est un endoscope.
